# EUROPÄISCHE PATENTSCHRIFT

(11) **EP 0 431 389 B1**
(45) Veröffentlichungstag und Bekanntmachung des Hinweises auf die Patenterteilung: **29.12.1993**
(21) Anmeldenummer: 90122138.2
(22) Anmeldetag: 20.11.1990
(51) Int. Cl.: C07C 317/36, C09B 29/08

(54) **Azofarbstoffe und Verfahren zur Herstellung von [5-Amino-2-(2-hydroxyethylamino)phenyl](2-hydroxyethyl)-sulfon**
Azo dyes and process for the manufacture of [5-amino-2-(2-hydroxyethylamino)phenyl](2-hydroxyethyl)sulfone
Colorants azoiques et procédé de préparation de la[5-amino-2-(2-hydroxyéthylamino)phenyl](2-hydroxyéthyl)sulfone

(30) Priorität: 02.12.1989 DE 3939966
(43) Veröffentlichungstag der Anmeldung: 12.06.1991
(73) Patentinhaber: BAYER AG, 51368 Leverkusen (DE)
(72) Erfinder: Herd, Karl-Josef, Dr., W-5068 Odenthal-Holz (DE)

(56) Entgegenhaltungen:
- EP-A- 0 153 599
- EP-A- 0 197 418
- EP-A- 0 311 893

## Beschreibung

Gegenstand der Erfindung ist ein Verfahren zur Herstellung von [5-Amino-2-(2-hydroxyethylamino)phenyl](2-hydroxyethyl)sulfon der Formel 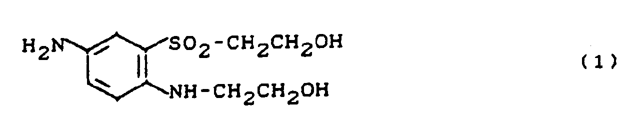
dadurch gekennzeichnet, daß man Benzothiazole der Formel 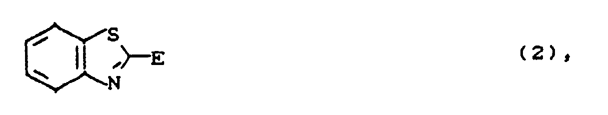
worin
E = H oder CH₃,
mit Ethylenoxid in wäßrigem Reaktiansmedium zu N-Formyl-bzw. N-Acetyl-N-(2-hydroxyethyl)anilin-Derivaten der Formel 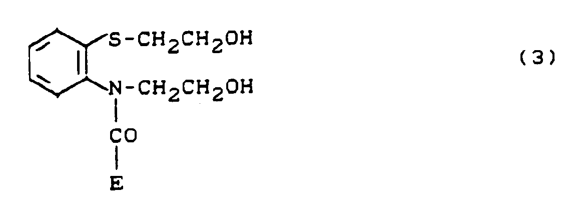
umsetz, anschließend zu dem Anilinderivat der Formel 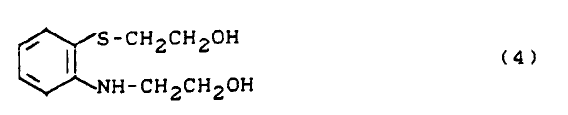
hydrolysiert, dieses mit einem diazotierten Amin D-NH₂ zu Monoazofarbstoffen der Formel 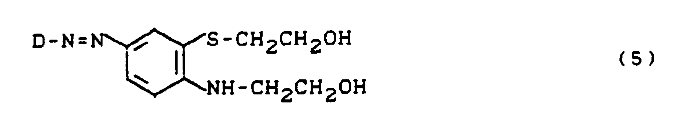
kuppelt, die Azofarbstoffe (5) zu Azo/Azoxyfarbstoffen der Formel 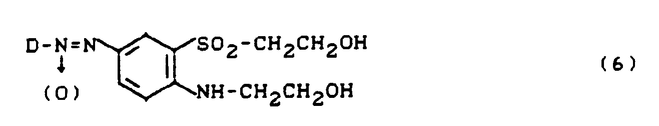
oxidiert und diese abschließend in an sich bekannter Weise reduktiv spaltet zu D-NH₂ und (1).

Gegenstand der Erfindung sind auch die neuen Azo- bzw. Azo/Azoxy-Farbstoffe der Formeln (5) und (6).

D steht für den Rest einer Diazokomponente, insbesondere aus der Benzol-, Naphthalin- oder Hetaryl-Reihe.

D kann durch übliche Substituenten substituiert sein, beispielsweise Cl, Br, F, C₁-C₄-Alkyl, C₁-C₄-Alkoxy, Carbalkoxy, Carboxy, Amino, Alkylamino, Dialkylamino, Sulfonamido, Alkylsulfonyl und insbesondere Sulfo, Es sind vorzugsweise solche Substituenten geeignet, die während der Oxidation und Reduktion keine Veränderung erfahren. Die Alkylgruppen können übliche Substituenten aufweisen,

Bevorzugt sind solche Azo- bzw. Azo/Azoxy-Farbstoffe der Formeln (5) und (6), worin der Rest D so gewählt ist, daß nach der Reduktion von (6) zu D-NH₂ und (1) die Diazokomponente D-NH₂ leicht abgetrennt und wieder in die Synthesefolge eingesetzt werden kann. Derartige Reste D sind beispielsweise 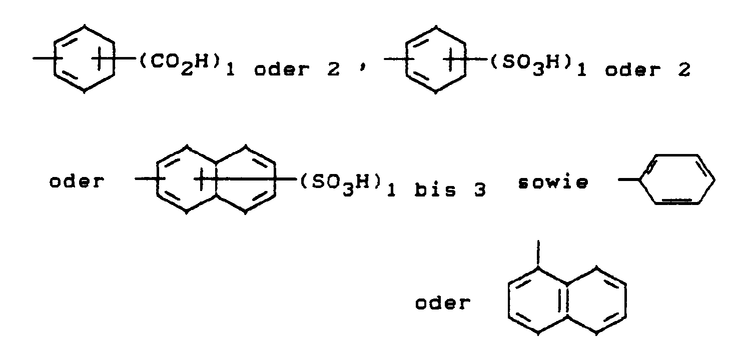
wobei die Benzol- bzw. Naphthalinringe weitere Substituenten aufweisen können, beispielsweise Cl, Br, C₁-C₄-Alkyl, C₁-C₄-Alkoxy oder Carboxy.

Sulfogruppenhaltige oder carboxygruppenhaltige Diazokomponenten bieten den Vorteil, daß die Oxidation sowie die Reduktion in wäßrigem Reaktionsmedium durchgeführt werden können.

Besonders bevorzugt sind Azo- bzw. Azo/Azoxy-Farbstoffe der Formeln 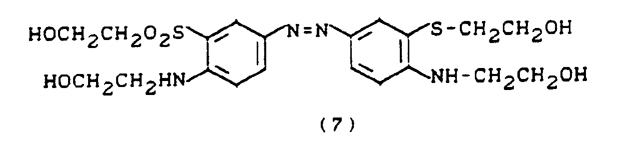
und 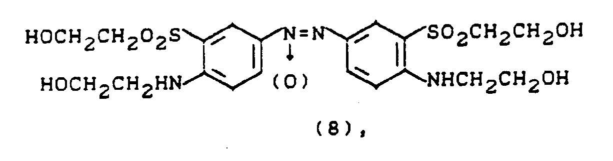
da die Reduktion von (8) einheitlich nur (1) liefert, (7) ist durch Diazotieren von (1) und Kuppeln auf (4) herstellbar,

Die Umsetzung der Benzothiazole (2) mit überschüssigem Ethylenoxid wird bevorzugt in Wasser bei Temperaturen zwischen 20 bis 120°C, vorzugsweise zwischen 40 bis 90°C, sowie im Autoklaven unter 0,2 bis 2,0 bar Druck durchgeführt. Das Ethylenoxid wird dabei so zudosiert, daß sich ein pH-Wert von 8 bis 12, vorzugsweise von 9,0 bis 10,5, einstellt. Vermutlich werden dabei die Zwischenstufen 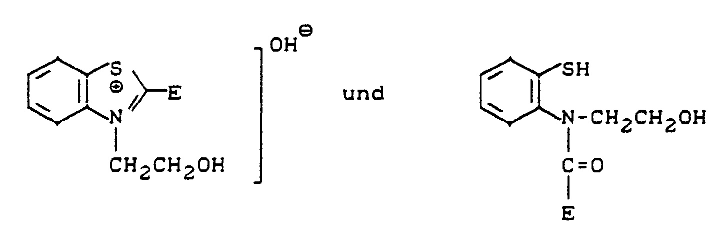
durchlaufen. Aus den Benzothiazolen (2) werden somit überraschenderweise durch einfache Ethylenoxidzugabe direkt wäßrige Lösungen von 2-(2-Hydroxyethylmercapto)-N-acyl-N-(2-hydroxyethyl)anilin der Formel (3) erhalten.

Die Ethoxylierungsreaktion kann auch in einem Reaktionsmedium, das sich aus Wasser und einem wassermischbaren organischen Lösemittel zusammensetzt, durchgeführt werden. Zusätze von Emulgatoren oder Phasentransferkatalysatoren können die Reaktion beschleunigen. Die Reaktionslösungen können im Prinzip direkt weiter umgesetzt werden.

Das N-Formyl- bzw. N-Acetylanilin-Derivat der Formel (3) ist sowohl unter sauren wie unter alkalischen Reaktionsbedingungen bei Temperaturen von 80 bis 120°C, gegebenenfalls unter Druck, hydrolysierbar. Die freie Anilinbase der Formel (4) scheidet sich bei pH-Werten oberhalb von 7, d.h. unter alkalischen Bedingungen, als ölige Phase ab.

Die Kupplung von (4) mit diazotierten Aminen D-NH₂ zu Farbstoffen der Formel (5) erfolgt am günstigsten bei 0 bis 30°C und einem pH-Bereich von 1,0 bis 5,0 im wäßrigen Medium. Auch hier kann sich der Einsatz von Emulgatoren oder Kupplungsbeschleunigern, wie z.B. Harnstoff, als vorteilhaft erweisen. Die Farbstoffe (5) fallen meist als kristalline Verbindungen an bzw. können als solche ausgesalzen werden. Da die Kupplungsreaktionen in den meisten Fällen relativ einheitlich verlaufen, kann oftmals direkt ohne Zwischenisolierung von (5) zu Azo/Azoxy-Farbstoffen der Formel (6) oxidiert werden. Als vorteilhaft hat sich dabei der Einsatz von Wasserstoffperoxid in Gegenwart katalytischer Mengen an Wolframaten erwiesen. Die Oxidation erfolgt in wäßrigem Medium bei 20 bis 100°C, vorzugsweise bei 40 bis 80°C und pH-Werten von 6 bis 8. Dabei werden als Zwischenstufen die entsprechenden Sulfoxidverbindungen der Formel 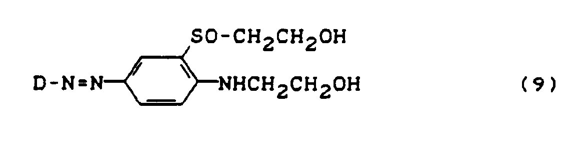
durchlaufen, was sich chromatographisch verfolgen läßt. Weitere geeignete Oxidationsmittel sind z.B. auch Perborate, Persulfate oder Persulfonsäuren.

Die Azo/Azoxy-Farbstoffe der Formel (6) sind als gelbe bis rote, kristalline, wasserlösliche Verbindungen isolierbar. Ihre Lösungen zeigen bei Protonierung eine deutliche bathochrome Verschiebung. In den meisten Farbstoffen (6) überwiegt der Azo-Anteil; in der Regel ist der Azoxy-Anteil auf 5 % beschränkt, wie z.B. in (8). Durch Umkristallisieren aus Wasser oder Alkohol kann der Azofarbstoff (8a) relativ einheitlich erhalten werden.

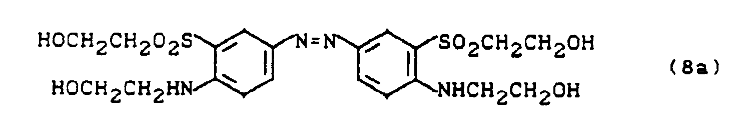

Die Reduktion von (6) bzw. (8) oder (8a) zu (1) und D-NH₂ kann nach Methoden erfolgen, wie sie von R. Schröter in Handbuch für präparative Methoden der organischen Chemie, Houben-Weyl, Band XI, Teil 1, Seite 522 bis 531, beschrieben werden. Als günstig erweist sich danach eine Reduktion mit Natriumdithionit oder Traubenzucker oder aber eine katalytische Reduktion mit Wasserstoff. Als Katalysatoren kommen dafür in erster Linie Raney-Nickel, Palladium-Kohle oder Platinverbindungen in Frage. Die Reduktionen werden bevorzugt in Wasser, Wasser-Alkohol-Gemischen oder Alkoholen bei 20 bis 80°C durchgeführt.

Im Falle von (8) bzw. (8a) resultiert eine einheitliche Lösung von (1). (1) kann in dieser Form direkt weiter umgesetzt werden, z.B. mit Chloranil kondensiert oder aber diazotiert werden, oder durch Aufkonzentrieren der Lösung als kristalline Substanz isoliert werden.

In allen anderen Fällen resultiert bei der Reduktion eine Lösung bzw. Suspension aus (1) und D-NH₂. Letzteres ist deshalb bereits als Diazokomponente so zu wählen, daß es nun nahezu quantitativ von (1) abgetrennt werden kann. Deshalb nimmt man zweckmäßig aromatische Aminocarbonsäuren oder Aminosulfonsäuren als Diazokomponenten, wie z.B.
2-Amino-benzoesäure
4-Amino-benzoesäure
2-Amino-benzolsulfonsäure
3-Amino-benzolsulfonsäure
4-Amino-benzolsulfonsäure
2-Amino-4-methylbenzolsulfonsäure
6-Amino-2-naphthalinsulfonsäure
7-Amino-1,3-naphthalindisulfonsäure
2-Amino-1,5-naphthalindisulfonsäure
4-Amino-1-naphthalinsulfonsäure
5-Amino-1-naphthalinsulfonsäure u.a.

Führt man die Reduktionen in rein wäßrigem Medium durch, so resultieren in diesen Fällen meist Lösungen, aus denen durch Sauerstellen und gegebenenfalls Aussalzen die Benzoesäuren oder die Aminosulfonsäuren als Betaine ausgeschieden und abgetrennt werden können. (1) verbleibt in der sauren wäßrigen Lösung und kann wie bereits beschrieben isoliert oder weiter umgesetzt werden.

Wählt man Anilin oder α-Naphthylamin als mögliche Diazokompomente, so ist nach Reduktion die Abtrennung derselben von (1) mittels Wasserdampfdestillation zu realisieren. Andere Wasserdampf-flüchtige Anilinderivate sind als Diazokomponenten ebenfalls geeignet.

Die Verbindung (1) findet beispielsweise Verwendung für die Herstellung interessanter Azofarbstoffe, wie Beispiel 17 aus DOS 3 512 340 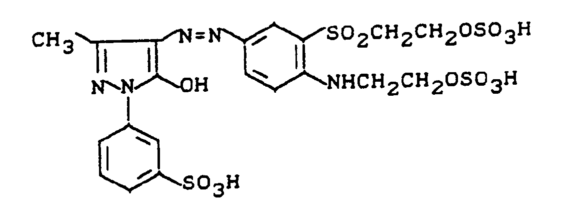
oder Beispiel 5 aus EP 279 351 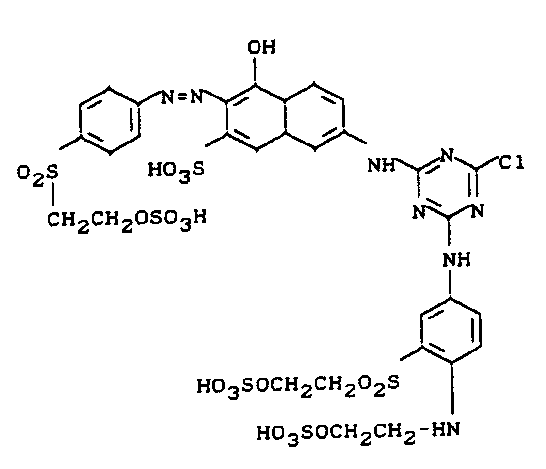
sowie für die Herstellung von Triphendioxazinfarbstoffen, wie Beispiel 1 aus EP 153 599

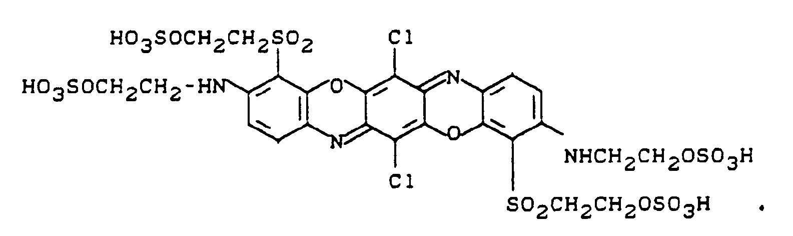

Azofarbstoffe der Formel (5) und (6) finden Verwendung zum Färben von Papier, Wolle, synthetischen Polyamidfasern oder Leder. Es werden klare, brillante gelbe Färbungen erhalten.

Die angegebenen Formeln für sulfogruppenhaltige und carboxygruppenhaltige Azofarbstoffe sind die der freien Säuren. Bei der Herstellung werden im allgemeinen die Salze erhalten, insbesondere die Alkalisalze, wie Natrium-, Kalium- oder Lithiumsalze.

### Beispiel 1

Eine Emulsion aus 135 g Benzothiazol und 1 g eines üblichen Emulgators in 500 ml Wasser wird auf 60°C erwärmt, und es wird für 1 Stunde Stickstoff durchgeleitet. Über einen Zeitraum von ca. 12 Stunden dosiert man dann insgesamt ca. 300 g Ethylenoxid so zu, daß ein pH-Wert zwischen 9,5 und 10,5 aufrechterhalten wird. Die Reaktion wird dünnschichtchromatographisch verfolgt. Sobald weniger als 1 % der Ausgangskonzentration des Benzothiazols nachweisbar sind, wird die Ethylenoxidzufuhr gestopt und die Reaktionslösung unter Durchleiten eines kräftigen Stickstoffstromes für weitere 2 Stunden auf 80° C erwärmt. Nachdem auf diese Weise das restliche Ethylenoxid entfernt wurde, kühlt man die ca. 800 ml Reaktionslösung auf Raumtemperatur ab. Die Reaktionslösung enthält neben Glykol und geringen Mengen Polyglykolen relativ einheitliches Hydroxyethylanilin-Derivat der Formel 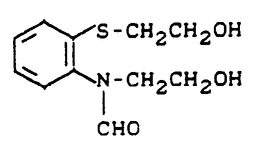
und kann direkt weiter umgesetzt werden.

Zur Charakterisierung des Produkts wird eine 80 ml-Probe der Lösung bei pH 7 extraktiv und destillativ aufgearbeitet. Es resultiert ein zähes farbloses Öl, dem aufgrund der ¹H-NMR- und IR-Daten die obige Struktur zugeordnet werden kann.
IR (Nujol-Verreibung): 1662 cm⁻¹ (CO-Schw.)
- 1H-NMR (d₆-DMSO): δ =: 3,05 (2H,m); 3,48 (2H,m); 3,60 (2H, m); 3,65 (2H, m); 4,68 (t, OH); 4,94 (t, OH); 7,15-7,50 (m, 4H); 8,00 (s, CHO).
- Massenspektrum: m/e =: 241 (M⁺, 45 %); 213 (M⁺-CO, 45 %); 182 (65 %); 164 (55 %); 136 (100 %).

### Beispiel 2

800 ml Reaktionslösung aus Beispiel 1 werden mit 200 ml einer 70 %igen Schwefelsäure versetzt und für eine Stunde auf 90-95° C erwärmt. Danach wird abgekühlt und unter Außenkühlung mit konzentrierter Natronlauge neutralisiert und anschließend auf pH 12,5 gestellt. Dabei scheidet sich ein farbloses Öl ab.

(Die Reaktionslösung aus Beispiel 1 kann aber auch alkalisch hydrolysiert werden, indem man mit Natronlauge auf pH 12,5-13,0 stellt und für 30 Minuten auf 80-85° C erwärmt)
Das Öl wird abgetrennt und als Hydroxyethylanilin der Formel 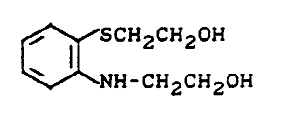
characterisiert.
- ¹H-NMR (d₆-DMSO): δ =: 2,77 (t, 2H); 3,18 (m, 2H); 3,42 (m, 2H); 3,60 (m, 2H); 4,81 (t, OH); 4,83 (t, OH); 5,46 (t, NH); 6,50-6,63 (m, 2H); 7,15 (m, 1H); 7,30 (m, 1H).

### Beispiel 3

Die Emulsion aus 149 g 2-Methylbenzothiazol, 1 g eines üblichen Emulgators und 500 ml Wasser wird in einem Druckbehälter auf 60°C erwärmt, und es wird für 1 Stunde Stickstoff durchgeleitet. Der Autoklav wird anschließend verschlossen und auf 80°C erwärmt. Es werden ca. 250 g Ethylenoxid über einen Zeitraum von 3-4 Stunden so aufgedrückt, daß ein Innendruck von 1,5 bar und ein pH-Wert zwischen 9,5 und 10,5 aufrechterhalten wird. Ist die Gesamtmenge an Ethylenoxid zugegeben, wird solange auf 80°C erwärmt, bis der Druck auf 0,1/-0,2 bar abgefallen ist. Es wird entspannt und bei 80°C durch Durchleiten eines kräftigen Stickstoffatomes das restliche Ethylenoxid entfernt. Die ca. 750 ml Reaktionslösung werden auf 20°C abgekühlt. 50 ml dieser farblosen Lösung werden am Rotationsdampfer aufkonzentriert und das zurückbleibende Öl säulenchromatographisch gereinigt, d.h. Glykol und Polyglykole abgetrennt. Man isoliert ein farbloses zähes Öl, das spektroskopisch als Verbindung der Formel 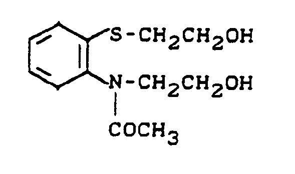
identifiziert wird.
- ¹H-NMR (d₆-DMSO): δ =: 1,65 (s, COCH₃); 3,04 (dt, 1H); 3,12 (t, 2H); 3,50 (dt, 2H); 3,63 (t, 2H); 4,08 (dt, 1H); 4,60 (breit, 2 OH); 7,17-7,50 (m, 4H).
- IR (Nujol): 1640 cm⁻¹: (CO-Schwingung)

Saure oder alkalische Hydrolyse dieser N-Acetyl-Anilinverbindungen liefert in Analogie zu Beispiel 2 die freie Base 2-(2-Hydroxyethylmercapto)-N-(2-hydroxyethyl)-anilin.

### Beispiel 4

23,6 g 7-Amino-1,3-naphthalindisulfonsäure-mono-Natriumsalz werden in 150 ml Wasser/50 g Eis und 20 ml konzentrierter Salzsäure angerührt und bei 5 bis 10°C tropfenweise mit 17 ml einer Natriumnitritlösung (300 g/l) versetzt. Die Diazotierung ist nach 1,5 Stunden beendet. Der geringe Überschuß an Nitrit wird durch Amidosulfonsäurezugabe zerstört. Die cremefarbene Suspension wird mit Sodalösung auf pH 2,5 gestellt. Dazu tropft man bei ca. 10°C eine auf pH 2,0 eingestellte Lösung von 15,0 g der freien Base aus Beispiel 2 in 100 ml Wasser. Es wird 4 bis 5 Stunden bei 10°C gerührt (pH 1,5-2,0). Zur Vervollständigung der Kupplungsreaktion wird der pH-Wert durch Zugabe von Natriumacetat auf 4,5 erhöht und erneut 2 Stunden nachgerührt. Es werden 40 g Kochsalz zugesetzt, 1 Stunde gerührt und der Niederschlag durch Absaugen isoliert. Nach dem Trocknen resultieren 48 g eines salzhaltigen Farbstoffes der Formel 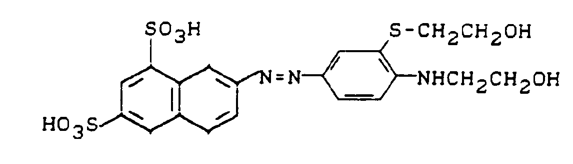
λmax = 454 nm (H₂O, pH 7-8)
- ¹H-NMR (d₆-DMSO) :δ =: 2,88 (t, 2H); 3,35 (t, 2H); 3,50 (t, 2H); 3,65 (m, 2H); 5,15 (breites s, 2 OH); 6,17 (t, NH); 6,85 (d, 1H); 7,83-8,07 (m, 4H); 8,16 (s, 1H); 8,30 (s, 1H); 9,30 (s, 1H).

### Beispiel 5

47 g des Farbstoffes aus Beispiel 4 werden in 250 ml Wasser bei pH 8-8,5 angerührt, mit 0,2 g Natriumwolframat versetzt und auf 70°C erwärmt. Man tropft 30 ml einer ca. 35 %igen wäßrigen Wasserstoffperoxidlösung zu- Die Temperatur sollte dabei zwischen 70 und 80°C betragen. Nach ca. 2 Stunden wird die Oxidation dünnschichtchromatographisch überprüft. Dabei ist auch das während der Oxidation durchlaufene Sulfoxid-Derivat nachweisbar. Es werden gegebenenfalls nochmals 10 bis 15 ml Wasserstoffperoxidlösung nachgesetzt, um auch das restliche Sulfoxid-Derivat zum Sulfon-Derivat aufzuoxidieren Es wird anschließend 2 Stunden bei 80° C gerührt, auf Raumtemperatur abgekühlt und mit verdünnter Schwefelsäure auf pH 0,5 bis 1,0 angesäuert. Der Farbstoff wird mit 35 g Kochsalz und 5 g Kaliumchlorid ausgesalzen und isoliert. Nach dem Trocknen erhält man 38 g eines salzhaltigen Farbstoffes der Formel

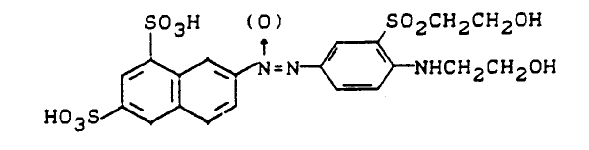

Der Azoxy-Anteil scheint aufgrund von DC und NMR nur wenige Prozente zu betragen.
λmax = 406 nm (H₂O, pH 7-8)
- ¹H-NMR (d₆-DMSO) :δ =: 3,42 (t, 2H); 3,55 (t, 2H); 3,68 (t, 2H); 3,75 (t, 2H); 5,0-5,3 (breit, 2 OH); 6,9 (breites s, NH); 7,13 (d, 1H); 7,98 (dd, 1H); 8,05-8,17 (m, 2H); 8,20 (s, 1H); 8,27 (d, 1H); 8,32 (d, 1H); 9,35 (d, 1H).

Variiert man nun in Beispiel 4 die Diazokomponente, so sind analog zur Kupplungsvorschrift (Beispiel 4) und Oxidationsvorschrift (Beispiel 5) weitere interessante Farbstoffe mit Azosulfid- und Azo/Azoxysulfon-Struktur herstellbar:

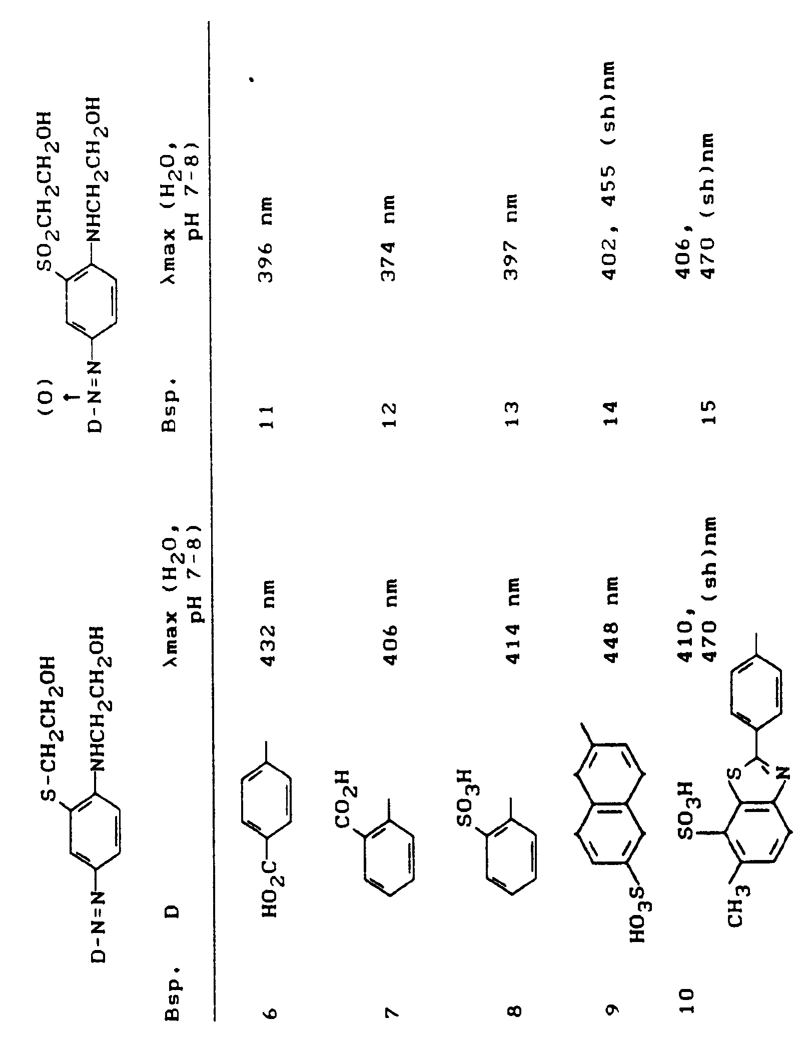

### Beispiel 16

12,5 g 4-Aminobenzolsulfonsäure werden analog zu Beispiel 4 diazotiert, und die resultierende Suspension mit Sodalösung auf pH 2,5 gestellt. Dazu tropft man bei 5 bis 10°C eine auf pH 2,0 eingestellte Lösung von 15,0 g der freien Base aus Beispiel 2 in 100 ml Wassern Es wird 5 Stunden bei pH 1,5 bis 2,5 und 10°C und anschließend 3 Stunden bei pH 3,5 bis 4,0 und 10°C gerührt. Der pH-Wert wird dabei mit Natriumacetatlösung erhöht. Nach erfolgter Kupplung wird der pH-Wert mit Sodalösung auf 8,0 eingestellt. Es resultiert eine klare Lösung des Azofarbstoffes der Formel

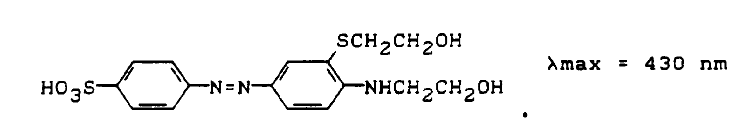

Die Lösung wird mit 0,2 g Natriumwolframat versetzt und auf 60°C erwärmt. Es werden über einen Zeitraum von 15 Minuten 35 ml einer 35 %igen wäßrigen Wasserstoffperoxidlösung zudosiert, und die Reaktion bei 70 bis 80° C gehalten. Die Oxidation zum Sulfon ist nach ca. 3 bis 4 Stunden beendet. Es wird auf 20° C abgekühlt, auf pH 6,0 gestellt und mit 45 g Kochsalz ausgesalzen. Nach weiteren 2 Stunden Rühren wird abgesaugt und getrocknet. Man erhält 32 g eines goldgelben Farbstoffpulvers der Struktur 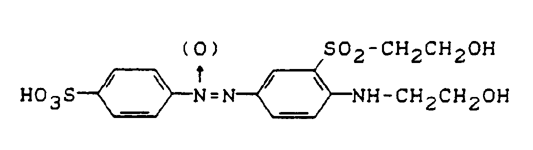
λmax = 396 nm (H₂O, pH 7-8)
- ¹H-NMR (d₆-DMSO): δ =: 3,38 (t, 2H); 3,50 (t, 2H); 3,60-3,75 (m, 4H); 5,0 (breites s, 2 OH); 6,94 (t, NH); 7,08 (d, 1H); 7,78 (m, 4H); 8,05 (dd, 1H); 8,17 (d, 1H).

### Beispiel 17

A) 35 g des Farbstoffes aus Beispiel 5 werden in 150 ml Wasser bei pH 7 gelöst, mit 0,2 g Raney-Nickel-Katalysator versetzt und im Autoklaven mit der 2,5-fachen äquimolaren Menge Wasserstoff reduziert. Der Ansatz kann sich dabei auf 40 bis 50°C erwärmen. Nach Abtrennen des Katalysators resultiert eine klare, blaß-bräunliche Lösung, aus der nach Sauerstellen auf pH 1,0, Aussalzen mit 10 g Kochsalz und Abkühlen auf 15° C die 7-Amino-1,3-naphthalindisulfonsäure in Form des mono-Natriumsalzes ausgeschieden werden kann. In Lösung verbleibt das gewünschte [5-Amino-2-(2-hydroxyethyl-amino)phenyl](2-hydroxyethyl)sulfon der Formel 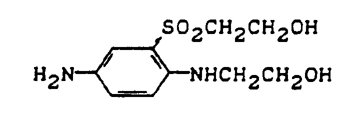
   Die Lösung kann nun, z.B. nach Neutralisation, direkt weiter umgesetzt werden. So z.B. mit Chloranil, wie es in EP 153 599 beschrieben ist. Sie kann aber auch nach Neutralisation am Rotationsverdampfer bis zur Trockene aufkonzentriert werden-Man erhält dann ein salzhaltiges Produkt, das durch Umkristallisieren aus Wasser oder Alkohol gereinigt werden kann und dann bei 130° C schmilzt.
B) Das gleiche Produkt bzw. die gleiche Lösung erhält man auch, wenn man 20 g des Farbstoffes aus Beispiel 16 in 100 ml Wasser bei pH 7-8 und 50°C innerhalb von 10 min mit 15,5 g Natriumdithionit umsetzt Die anfangs goldgelbe Lösung wird entfärbt, Man kühlt ab auf 20°C und stellt die Lösung mit Salzsäure auf pH 1,5. Nach Zugabe von 20 g Kochsalz rührt man ca. 4 bis 5 Stunden bis die 4-Aminobenzolsulfonsäure vollkommen ausgefällt ist. Nach Filtration resultiert eine Lösung von relativ einheitlichen [5-Amino-2-(2-hydroxyethylamino)-phenyl](2-hydroxyethyl)sulfon.

Dieses wichtige Zwischenprodukt wird ebenfalls erhalten, wenn man die Azo/Azoxyfarbstoffe der Beispiele 11 bis 16 analog zu Beispiel 17 A katalytisch hydriert und die ebenfalls gebildete Diazokomponente D-NH₂ wie beschrieben abtrennt.

### Beispiel 18

18,8 g [5-Amino-2-(2-hydroxyethylamino)phenyl](2-hydroxyethyl)sulfon werden in 150 ml Wasser, 50 g Eis und 25 ml Salzsäure gelöst und bei 0 bis 3° C mit 17 ml einer Natriumnitritlösung (300 g/l) diazotiert. Die Diazotierung ist nach 30 Minuten beendet; es resultiert eine Lösung.

15,2 g der freien Base aus Beispiel 2 werden in 50 ml Wasser suspendiert und durch Zugabe von 25 ml Essigsäure gelöst. Diese Lösung fügt man zur fertigen Diazotierungslösung und erhöht den pH-Wert durch Zugabe von Natriumacetatlösung auf 3,5 bis 4,0. Mit Eis hält man die Reaktionstemperatur bei 0-5°C und rührt für 8 Stunden bei pH 3,5 bis 4,0. Es scheidet sich ein kristalliner gelber Niederschlag ab, der abgesaugt, mit 50 ml Wasser gewaschen und getrocknet wird. Man erhält 31,5 g des Azofarbstoffes der Formel 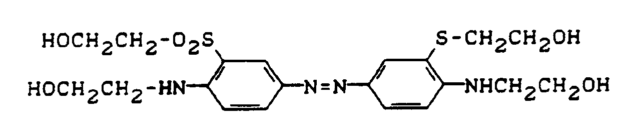
der aus Wasser oder Alkohol umkristallisierbar ist und bei 177-178°C schmilzt.
λmax = 458 nm (H₂O, pH 7-8)
- ¹H-NMR (d₆-DMSO) :δ =: 2,87 (t, 2H); 3,35 (m, 4H); 3,50 (m, 4H); 3,60-3,75 (m, 6H); 4,95 (breites 5, 4 OH); 6,13 (t, NH); 6,77 (t, NH); 6,80 (d, 1H); 7,05 (d, 1H); 7,75 (dd, 1H); 7,90 (d, 1H); 7,95 (dd, 1H); 8,07 (d, 1H).

### Beispiel 19

30 g des Azofarbstoffes aus Beispiel 18 werden in 150 ml Wasser suspendiert, die Mischung auf pH 8 gestellt und nach Zugabe von 0,2 g Natriumwolframat auf 60°C erwärmt (es kann aber auch anstelle des isolierten Farbstoffs die Kupplungsmischung aus Beispiel 18 direkt eingesetzt werden). Es werden langsam 30 ml einer 35 %igen Wasserstoffperoxidlösung zugetropft. Die Reaktion verläuft schwach exotherm. Bei 75° C resultiert eine klare orangerote Lösung. Es wird eine Stunde bei 75 bis 80° C nachgerührt und dann auf Raumtemperatur abgekühlt. Dabei kristallisiert der Azofarbstoff in goldgelben Kristallen aus. Zur Vervollständigung der Fällung wird mit 10 g Kaliumchlorid versetzt und auf 0° C abgekühlt. Man saugt ab, wäscht mit wenig Wasser nach und trocknet bei 60° C im Vakuum. Es resultieren 28,2 g Azofarbstoff der Formel 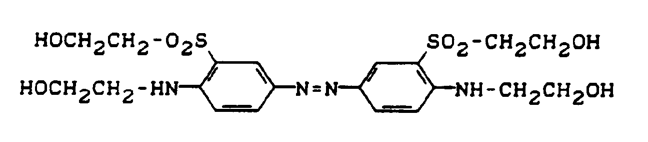
der aus Wasser oder Alkohol umkristallisiert werden kann und einen Schmelzpunkt von 200° C aufweist.
λmax = 442 nm (H₂O, pH 7-8)
- ¹H-NMR (d₆-DMSO): δ =: 3,38 (t, 4H); 3,50 (t, 4H); 3,62-3,75 (m, 8H); 4,93 (t, 2 OH); 4,97 (t, 2 OH); 6,95 (t, 2 NH); 7,06 (d, 2H); 8,01 (dd, 2H); 8,12 (d, 2H).

### Beispiel 20

25,8 g des Azofarbstoffes aus Beispiel 19 werden in 200 ml Wasser suspendiert, mit 0,2 g Raney-Nickel-Katalysator versetzt und im Autoklaven mit der 2-fachen äquimolaren Menge an Wasserstoff hydriert. Die Reaktion verläuft schwach exotherm; es wird eine Temperatur von 35 bis 40° C erreicht. Man erhält eine farblose Reaktionslösung, wenn man bei 40° C den Katalysator durch Filtration abtrennt. Aufkonzentrieren der Lösung am Rotationsverdampfer liefert 26 g einheitliches kristallines [5-Amino-2-(2-hydroxyethylamino)phenyl](2-hydroxy-ethyl)sulfon der Formel

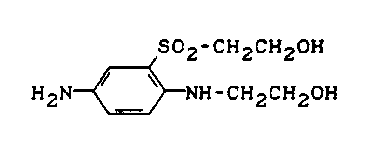

### Beispiel 21

Kuppelt man diazotiertes Anilin in Analogie zu Beispiel 16 in wäßrigem Medium auf die freie Base 2-(2-Hydroxyethylmercapto)-N-(2-hydroxyethyl)anilin und oxidiert die erhaltene Azoverbindung bei 60-80°C mit Wasserstoffperoxidlösung, so resultiert ein kristallines Azosulfon der Formel

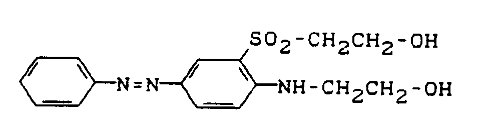

Die goldgelbe Verbindung (λₘₐₓ = 392 nm (H₂O, pH 7-8)) schmilzt bei 126°C.
- ¹ H-NMR (d₆-DMSO): δ =: 3,38 (m, 2H); 3,50 (m, 2H); 3,68 (m, 2H); 3,73 (m, 2H); 4,90 (t, OH); 4,95 (t, OH); 6,92 (t, NH); 7,08 (d, 1H); 7,45-7,58 (m, 3H); 7,80 (d, 1H); 7,86 (d, 1H); 8,04 (dd, 1H); 8,18 (d, 1H).

### Beispiel 22

20 g des Azofarbstoffes aus Beispiel 21 werden in 200 ml Wasser suspendiert, mit 0,2 g Raney-Nickel-Katalysator versetzt und im Autoklaven mit der 2-fachen äquimolaren Menge an Wasserstoff hydriert. Es wird dabei eine Temperatur von 40°C erreicht Nach Reaktionsende wird der Katalysator abgetrennt und das gebildete Anilin durch eine Wasserdampfdestillation restlos aus der Lösung entfernt. Die verbleibende Lösung, die [5-Amino-2-(2-hydroxyethyl-amino)phenyl](2-hydroxyethyl)sulfon enthält, kann direkt für weitere Umsetzungen, wie z.B. die Kondensation mit Chloranil eingesetzt werden (vgl. Beispiel 17). Obige Reduktion ist z.B. auch in Methanol oder in einem Methanol-Wasser-Gemisch durchführbar.

## Patentansprüche

1. Verfahren zur Herstellung von [5-Amino-2-(2-hydroxyethylamino)phenyl](2-hydroxyethyl)sulfon der Formel 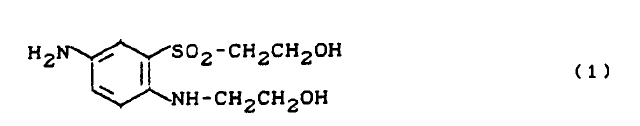 dadurch gekennzeichnet, daß man Benzothiazole der Formel 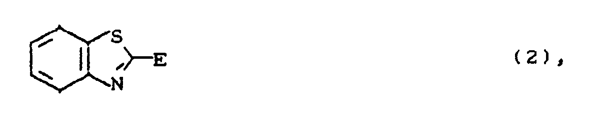 worin
E = H oder CH₃,
mit Ethylenoxid in wäßrigem Reaktionsmedium zu N-Formyl-bzw. N-Acetyl-N-(2-hydroxyethyl)anilin-Derivaten der Formel 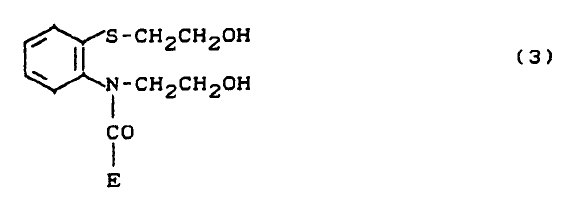 umsetzt, anschließend zu dem Anilinderivat der Formel 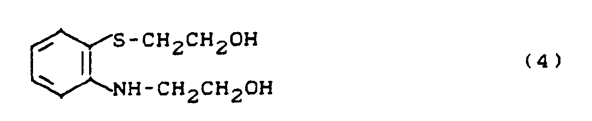 hydrolysiert, dieses mit einem diazotierten Amin D-NH₂ zu Monoazofarbstoffen der Formel 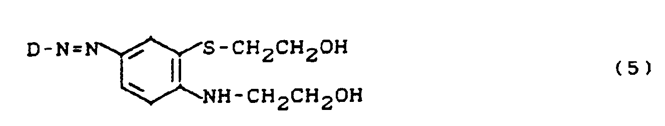 kuppelt, die Azofarbstoffe (5) zu Azo/Azoxyfarbstoffen der Formel 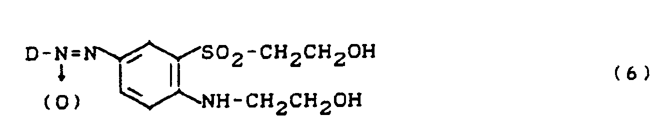 oxidiert und diese abschließend in an sich bekannter Weise reduktiv spaltet zu D-NH₂ und (1), worin D = Rest einer Diazokomponente.

2. Verfahren nach Anspruch 1, dadurch gekennzeichnet, daß Amine D-NH₂ verwendet werden, die Sulfo- und/oder Carboxygruppen aufweisen oder D-NH₂ für Anilin oder α-Naphthylamin steht.

3. Verfahren zur Herstellung des Sulfons der Formel 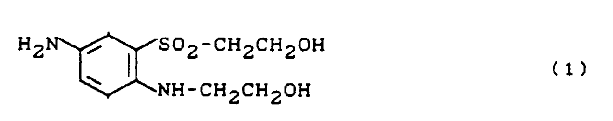 dadurch gekennzeichnet, daß man einen Azo/Azoxyfarbstoff der Formel 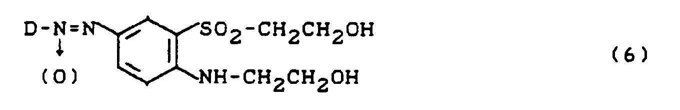 reduktiv spaltet.

4. Azofarbstoffe der Formel 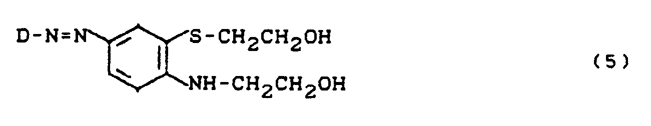 worin
D = Rest einer Diazokomponente, insbesondere aus der Benzol-, Naphthalin- oder Hetaryl-Reihe.

5. Azo/Azoxyfarbstoffe der Formel 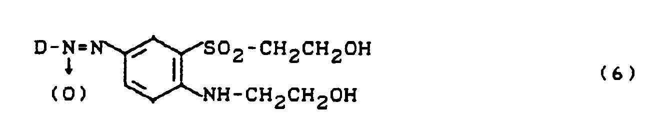 worin
D = Rest einer Diazokomponente, insbesondere aus der Benzol-, Naphthalin- oder Hetarylreihe.

6. Azofarbstoff der Formel

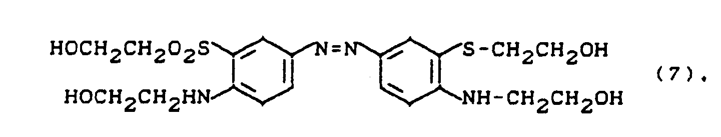

7. Azo/Azoxyfarbstoff der Formel

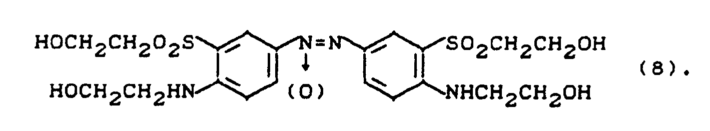

## Claims

1. Process for the preparation of [5-amino-2-(2-hydroxyethylamino)phenyl] (2 -hydroxyethyl) sulphone of the formula 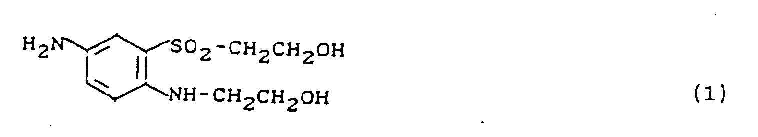 characterized in that benzothiazoles of the formula 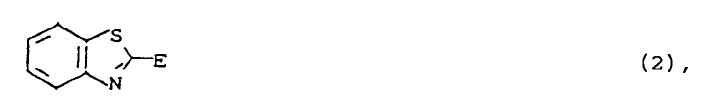 in which
E is H or CH₃,
are reacted with ethylene oxide in an aqueous reaction medium to give N-formyl- or N-acetyl-N-(2-hydroxyethyl)-aniline derivatives of the formula 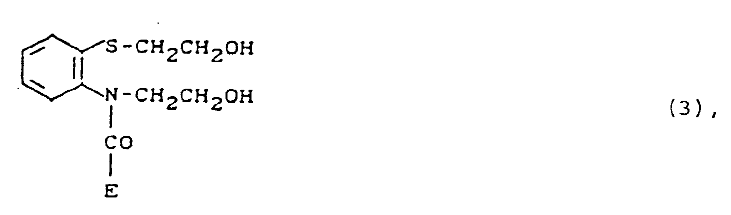 which are then hydrolysed to give the aniline derivatives of the formula 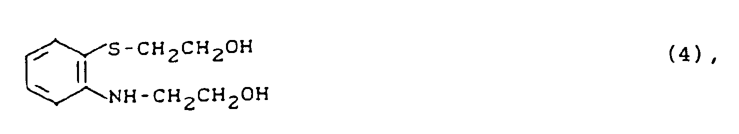 which are then coupled onto a diazotized amine D-NH₂ to give monoazo dyestuffs of the formula 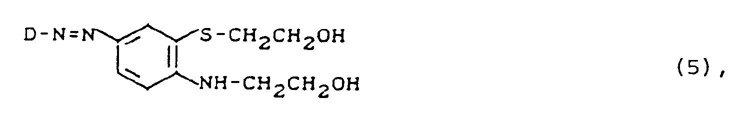 the azo dyestuffs (5) are then oxidized to give azo/azoxy dyestuffs of the formula 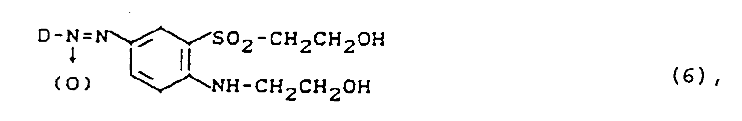 which are then cleaved reductively in a manner known per se to give D-NH₂ and (1), in which D is the radical of a diazo component.

2. Process according to Claim 1, characterized in that amines D-NH₂ are used which contain sulpho and/or carboxyl groups or D-NH₂ represents aniline or α-naphthylamine.

3. Process for the preparation of the sulphone of the formula 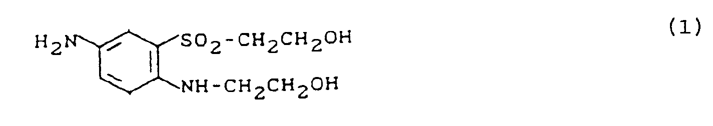 characterized in that an azo/azoxy dyestuff of the formula 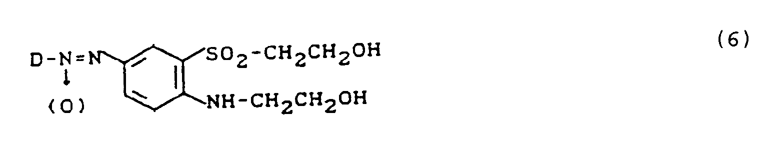 is cleaved reductively.

4. Azo dyestuffs of the formula 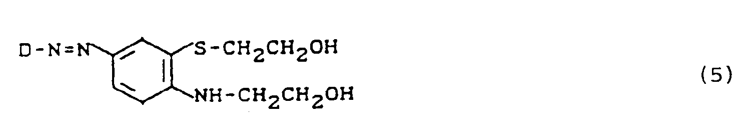 in which D is the radical of a diazo component, in particular from the benzene, naphthalene or hetaryl series.

5. Azo/azoxy dyestuffs of the formula 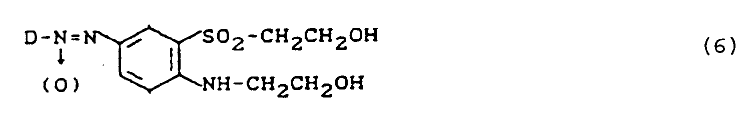 in which
D is the radical of a diazo component, in particular from the benzene, naphthalene or hetaryl series.

6. Azo dyestuff of the formula

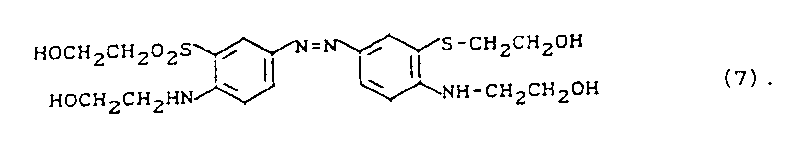

7. Azo/azoxy dyestuff of the formula

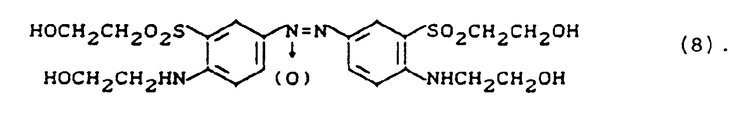

## Revendications

1. Procédé de préparation de la [5-amino-2-(2-hydroxyéthylamino)-phényl]-(2-hydroxyéthyl)-sulfone de formule 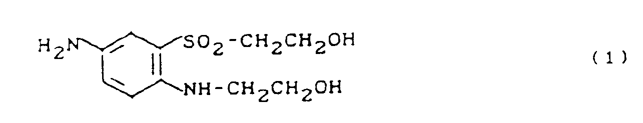 caractérisé en ce que l'on fait réagir des benzothiazoles de formule 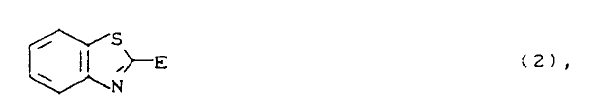 dans laquelle
E = H ou CH₃,
avec l'oxyde d'éthylène dans un milieu de réaction aqueux, ce qui donne respectivement les dérivés de N-formyl- et de N-acétyl-N-(2-hydroxyéthyl)-aniline de formule 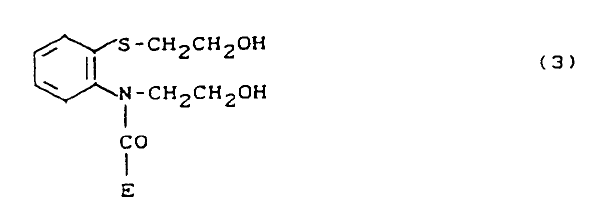 qu'on convertit ensuite par hydrolyse en le dérivé d'aniline de formule 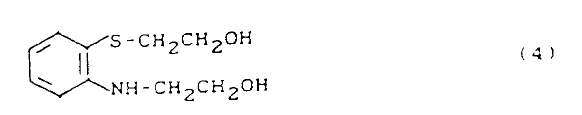 qu'on copule avec le diazo d'une amine D-NH₂, ce qui donne les colorants monoazoïques de formule 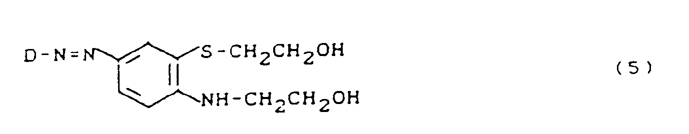 qu'on oxyde en les colorants azo/azoxyliques de formule 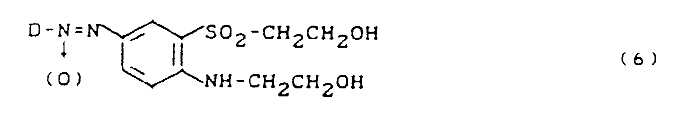 lesquels sont ensuite soumis de manière connue en soi à scission réductive en D-NH₂ et (1), D représentant le radical d'un composant diazotable.

2. Procédé selon la revendication 1, caractérisé en ce que l'on utilise des amines D-NH₂ contenant des groupes sulfo et/ou carboxy, ou bien D-NH₂ représente l'aniline ou l'α-naphtylamine.

3. Procédé de préparation de la sulfone de formule 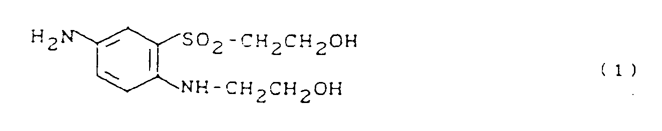 caractérisé en ce que l'on soumet un colorant azo/azoxylique de formule 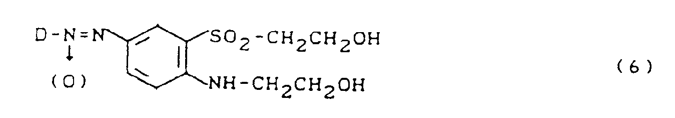 à scission réductive.

4. Colorants azoïques de formule 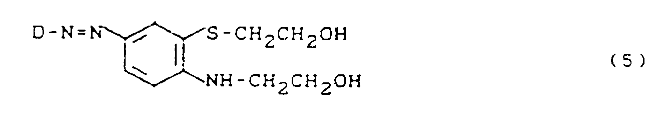 dans laquelle
D représente le radical d'un composant diazotable appartenant en particulier à la série benzénique, à la série naphtalénique ou à la série hétéroarylique.

5. Colorants azo/azoxyliques de formule 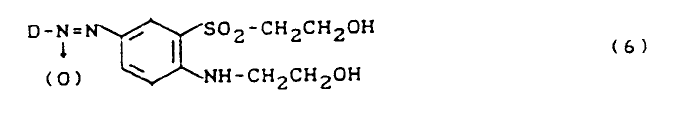 dans laquelle
D représente le radical d'un composant diazotable appartenant en particulier à la série benzénique, naphtaléniquc ou hétéroarylique.

6. Colorant azoïque de formule

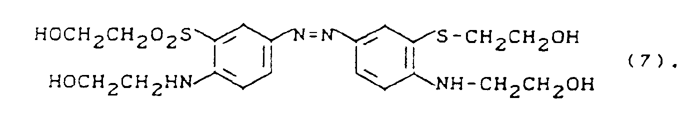

7. Colorant azo/azoxylique de formule
